# EUROPEAN PATENT SPECIFICATION

(11) **EP 2 848 261 B1**
(45) Date of publication and mention of the grant of the patent: **20.04.2016**
(21) Application number: 14184446.4
(22) Date of filing: 11.09.2014
(51) Int. Cl.: A61K 45/06, A61K 31/192, A61K 31/704, A61K 47/44, A61K 9/20

(54) **Pharmaceutical formulations comprising a muscle relaxant and an analgesic combination**
Pharmazeutische Formulierungen mit einem Muskelrelaxans und einer Schmerzmittelkombination
Formulations pharmaceutiques comprenant un relaxant des muscles et une combinaison d'un analgésique

(30) Priority: 12.09.2013 TR 201310747; 12.09.2013 TR 201310731; 12.09.2013 TR 201310746
(43) Date of publication of application: 18.03.2015
(73) Proprietor: Sanovel Ilac Sanayi ve Ticaret A.S., 34460 Istanbul (TR)
(72) Inventor: Türkyilmaz, Ali, 34460 Istanbul (TR); Pehlivan Akalin, Nur, 34460 Istanbul (TR)
(74) Representative: Sevinç, Erkan

(56) References cited:
- WO-A1-2011/112161
- WO-A1-2012/144964
- WO-A2-2010/103544

## Description

### Technical Aspect

This invention is a novel pharmaceutical formulation comprising a thiocolchicoside and an analgesic combination for oral administration with anti-inflammatory, analgesic, myorelaxant activity and methods of its manufacture. The pharmaceutical composition of the present invention is administered orally in tablet, multilayer tablet, multicoated tablet and capsule form.

More particularly, this present invention relates to a pharmaceutical formulation comprising an immediate release phase comprising an effective amount of thiocolchicoside or a pharmaceutically acceptable salt thereof, and first sustained release phase comprising an effective amount of thiocolchicoside or a pharmaceutically acceptable salt thereof and glyceryl behenate as release rate controlling agent; and second sustained release phase comprising an effective amount of an analgesic or a pharmaceutically acceptable salt thereof.

### Background of the Invention

As it is known, colchicine is a pseudoalcaloid that has been widely used for a long time in therapy for the treatment of gout. The use of 3- demethyl- thiocolchicine glucoside, known as thiocolchicoside, is also widespread in therapy for treating contractures and inflammatory conditions that affect the muscular system (Ortopedia e Traumatologia Oggi XII, n. 4, 1992).

Thiocolchicoside has been claimed to possess GABA-mimetic and glycinergic actions. In other words we can say that thiocolchicoside is a gamma-aminobutiric acid receptor agonist. Its chemical structure is shown in Formula 3.

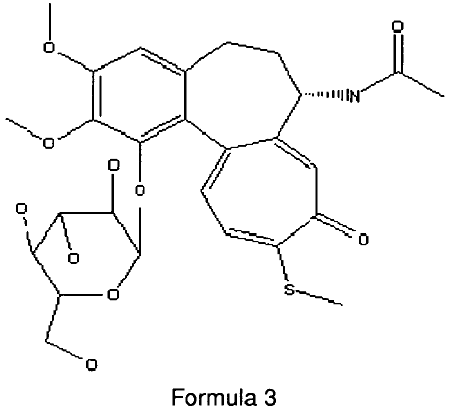

It has recently been shown that thiocoichicoside's activity can be ascribed to its ability to interact with the strychnine-sensitive glycine receptors and therefore that compounds endowed with glycino-mimetic activity can be used in the rheumatologic- orthopedic field for their muscle relaxant properties.

Thiocolchicoside is known a muscle relaxant agent used in the treatment of painful muscle spasms or spasticity occurring in musculoskeletal and neuromuscular disorders and for treating contractures and inflammatory conditions that affect the muscular system.

The usual initial dose of thiocolchicoside is 16 mg daily by mouth. It has also been given intramuscularly, in doses up to 8 mg daily, or applied as cream or ointment (Sean C Sweetman, Martindale The Complete Drug Reference, thirty-fifth edition 2007, Vol. 1, page 1738).

Analgesics are drugs used to relieve pain and known as "pain killers". Pain is one of the most common symptoms, and one of the most frequent reasons why people seek medical care. Analgesic drugs are used for management of the pain by acting in various ways on the peripheral and central nervous systems. In prior art, there are several example of pharmaceutical formulations in which a muscle relaxant agent that is combined with an analgesic for the treatment of pain, spasticity, inflammatory symptoms and painful muscle spasms.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, undesirable drug interactions or new side effects. More specifically, in the area of analgesia there are drug combinations that are contraindicated for some or all of these very same reasons.

The main challenges of combining two or more molecules in the same pharmaceutical form are (a) to ensure the chemico-physical compatibility between the different active ingredients and/or between the active ingredients and the excipients used; and (b) to ensure the therapeutical compatibility between the two active ingredients regarding their pharmacokinetic and/or pharmaceutical properties in order that the posology of the combined composition allows to obtain safe and efficient plasma levels of both pharmacological agents.

Conventional analgesic and myorelaxant therapy generally involves administration of a pharmaceutical composition containing one or more different analgesic and muscle relaxant drugs. However, not all analgesic and muscle relaxant drug combinations are more suitable, in terms of safety or reduced side effects; greater convenience; or have higher levels of patient compliance due to the simplified dosage schedule as compared to those of conventional formulation.

Additionally, novel pharmaceutical composition in the form of a tablet or a capsule administered orally may provide a significant advance in the available treatments. Such combination therapy may also provide therapeutic improvements owing to the potential synergistic effect provided by the combination.

WO 1998/052545 A1 discloses a pharmaceutical composition comprising a combination of a therapeutically effective amount of flurbiprofen with (a) a therapeuticaliy effective amount of one or more active ingredients selected from an antihistamine, a cough suppressant, a decongestant, an expectorant, a muscle relaxant, a centrally acting analgesic, a local anaesthetic, an antibacterial compound, an antiviral compound, an antibiotic compound, an antifungal compound, minerals and vitamins and/or (b) a burn-masking amount of an agent which has a warming effect on the mucosa of the throat said composition being in the form of a masticable or suckable solid dosage form or a liquid or a spray.

WO 2007/072503 A2 discloses a pharmaceutical compositions comprising at least one analgesic and antiinflammatory compound(s) that inhibits both COX and LOX as active agent in combination with at least one another active agent optionally with one or more other pharmaceutically acceptable excipient(s).

WO 1986/003681 A1 discloses a pharmaceutical composition for use in the treatment of a skeletal muscle disorder in a mammal, said composition comprising: an effective amount of a skeletal muscle relaxant, and an analgesically effective amount of a nonsteroidal anti-inflammatory drug, wherein said nonsteroidal anti-inflammatory drug comprises a propionic acid derivative, acetic acid derivative, fenamic acid derivative, biphenylcarboxylic acid derivative or an oxicam, or the pharmaceutically acceptable salts thereof.

Taking consideration of the prior art, several pharmaceutical compositions comprising combinations of a muscle relaxant and a conventionl analgesic has been formulated. But, an immediate release phase of thiocolchicoside and first sustained release phase of thiocolchicoside comprising glyceryl behenate as release rate controlling agent have not been previously combined with second sustained release phase of an analgesic in a pharmaceutical composition for the treatment of inflammatory, pain and musculoskeletal diseases in an effective and safe way.

Accordingly, there remains a need to develop a pharmaceutical formulation comprising a) an immediate release phase of thiocolchicoside, and b) first sustained release phase of thiocolchicoside comprising glyceryl behenate as release rate controlling agent, and c) second sustained release phase of an analgesic; that provides once or twice daily dosing for effective management of pain, spasticity, inflammatory symptoms and painful muscle spasms, while providing an improved side effect profile and increased patient compliance.

### Objects of the Invention

The main object of the present invention is to provide a pharmaceutical formulation comprising a) an immediate release phase comprising an effective amount of thiocolchicoside or a pharmaceutically acceptable salt thereof, b) first sustained release phase comprising an effective amount of thiocolchicoside or a pharmaceutically acceptable salt thereof and glyceryl behenate as release rate controlling agent, and c) second sustained release phase comprising an effective amount of an analgesic or pharmaceutically acceptable salt thereof; for the treatment of pain, spasticity, inflammatory symptoms and painful muscle spasms by eliminating all the aforementioned drawbacks and providing additional advantages to the respective technical field.

Another object of the present invention is to provide a pharmaceutical formulation comprising a compatible drug combination of thiocolchicoside or a pharmaceutically acceptable salt thereof and an analgesic or a pharmaceutically acceptable salt thereof that are combined into safe and efficacious dosage forms.

Another object of the present invention is to provide a pharmaceutical formulation that has a simplified dosage regimen in comparison with conventional dosage form comprising thiocolchicoside and an analgesic combination.

Another object of the present invention is to provide a pharmaceutical formulation that allows to obtain safe and efficient plasma levels of both pharmacological agents over an extended period of time that also results in increased patient compliance.

Another object of the present invention is to provide a pharmaceutical formulation that can be produced with good physical properties properties regarding hardness, friability, weight variation, thickness and drug content uniformity.

Another object of the present invention is to provide a process for the manufacture of said sustained release pharmaceutical formulation comprising thiocolchicosdie and an analgesic combination.

### Detailed Description of the Invention

### List of Figures

**Figure 1** shows multilayered tablet formulation of the invention comprising first sustained release phase of thiocolchicoside (a), an immediate release phase of thiocolchicoside (c), and second sustained release phase of an analgesic (b).
**Figure 2** shows a multilayered tablet in which there is a barrier layer (d) present between two layers to separate them.
**Figure 3** shows a multilayered tablet in which there are two barrier layers (e), (f) each of which present between two layers to separate them.
**Figure 4** shows multicoated tablet that comprises a core consisting of two layers, one is first sustained-release phase of thiocolchicoside (g), the other is second sustained-release phase of flurbiprofen (h); the coating layer comprising thiocolchicoside which is released immediately (i); and a polymer coating layer (i) including conventional coating polymers.
**Figure 5** shows a multicoated tablet that comprises a core consisting of three layers, one of which comprises first sustained-release phase of thiocolchicoside (k), and other layer comprises second sustained-release phase of flurbiprofen (j), wherein there is also a barrier layer (n); and a polymer coating layer including conventional coating polymers (I); and a coating layer comprising thiocolchicoside which is released immediately (m).

This invention is a novel pharmaceutical formulation of thiocolchicoside in combination with an analgesic for oral administration with anti-inflammatory, analgesic, myorelaxant activity and methods of its manufacture.

The term "sustained release phase" refers to any pharmaceutical formulation that maintain constant levels of a drug in the patient's bloodstream by releasing the drug over an extended period of time. Sustained release phase are formulated to release the active ingredient gradually and predictably over a 12-hour to 24-hour period.

The term "immediate release phase" refers to any pharmaceutical formulations that disintegrate rapidly after administration with enhanced rate of dissolution and get dissolved to release the medicaments.

The term "release rate controlling agent" refers to an excipient in the final dosage form whose primary function is to modify the duration of release of the active drug substance from the dosage form.

The term "dosage form" refers to any form of the formulation that contains the active agent(s) in an amount sufficient to achieve a therapeutic effect with a single administration.

The term "active agent" refers to the agent, ingredient, drug or other substance that provides some beneficial pharmacological effect for the treatment. Within the scope of the invention, the active agent is thiocolchicoside or a pharmaceutically acceptable salt thereof and an analgesic or a pharmaceutically acceptable salt thereof.
The term "pharmaceutically acceptable excipient" refers to any ingredient having no therapeutic activity and being nontoxic and thus suitable as an excipient.

The present invention relates to a pharmaceutical formulation comprising a) an immediate release phase comprising an effective amount of thiocolchicoside or a pharmaceutically acceptable salt thereof, and b) first sustained release phase comprising an effective amount of thiocolchicoside or a pharmaceutically acceptable salt thereof and glyceryl behenate as release rate controlling agent, and c) second sustained release phase comprising an effective amount of an analgesic or pharmaceutically acceptable salt thereof.

This invention is a pharmaceutical formulation comprising thiocolchicoside and an analgesic combination for oral administration with anti-inflammatory, analgesic, myorelaxant activity and methods of its manufacture.

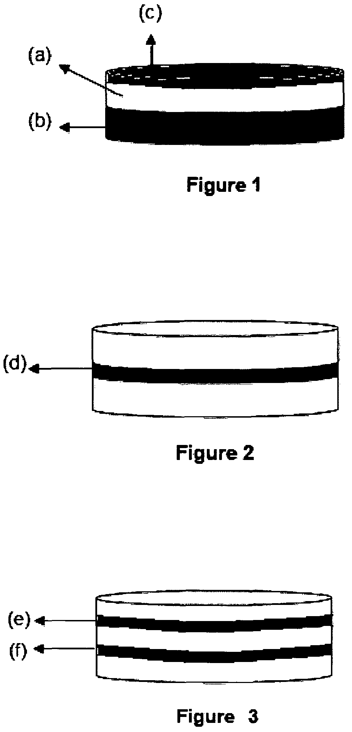

One aspect of the present invention, the pharmaceutical formulation is in the form of a tablet comprising one layer comprising an immediate release phase comprising thiocolchicoside, and one layer first sustained release phase comprising thiocolchicoside and glyceryl behenate as release rate controlling agent, and another layer comprising second sustained release phase comprising an analgesic *(**Figure 1**).*

In other words, the pharmaceutical formulation of the present invention comprising:
- the immediate release phase comprising thiocolchicoside or a pharmaceutically acceptable salt and at least one pharmaceutically acceptable excipient,
- the first sustained release phase comprising thiocolchicoside or a pharmaceutically acceptable salt, glyceryl behenate as release rate controlling agent, and at least one pharmaceutically acceptable excipient, and
- the second sustained release phase comprising an analgesic, in particular flurbiprofen, or a pharmaceutically acceptable salt and at least one pharmaceutically acceptable excipient.

Thiocolchicoside is a rapidly and highly soluble active ingredient and almost completely absorbed after oral administration. Because of the mentioned properties of thiocolchicoside, a conventional tablet or a capsule formulation of thiocolchicoside is required to be administered three-times daily or four times daily to maintain the therapeutic effect over an extended period of time. Therefore, thiocolchicoside that is administered in the form of a conventional tablet or a capsule causes plasma concentration level to be initially very high, followed by a rapid decline, and this results in a transient dose dumping, followed by a long period of under dosing. So, it leads to fluctuations of drug concentration in the blood plasma and undergoes repeated chemical imbalances, which might be detrimental to the patient's health. Because of this reason, thiocolchicoside can not be combined easily with every analgesic into a pharmaceutical dosage form thereby resulting in incompatible drug combinations that leads to increased side effects or new side effects, undesirable drug interactions and low patient compliance due to the complicated dosage schedule.

Suprisingly, it is found that when glyceryl behenate is used as release rate controlling agent in the first sustained release phase of thiocolchicoside, a compatible combination of thiocolchicoside and the analgesic is achieved in terms of reliability and providing high patient compliance due to the simplified dosage regimen since incremental release of thiocolchicoside is provided to be in controlled manner to make the plasma concentration level stable by maintaining release of thiocolchicoside in the blood stream over an extended period of time. Additionally, sustained release of the analgesic also makes the plasma concentration level of the analgesic safe to combine with thiocolchicoside. Therefore, pharmaceutical formulations of thiocolchicoside and analgesic show synergistic effect in terms of compatible drug combination and high patient compliance.

According to the present invention, the pharmaceutical formulation also comprises an immediate release phase of thiocolchicoside besides the first sustained release phase of thiocolchicoside and the second sustained release phase of the analgesic. The immediate release phase of thiocolchicoside releases rapidly thiocolchicoside after administration to provide sufficient blood concentration in a short time for starting therapeutic effect, and the first sustained release phase of thiocolchicoside provide release of thiocolchicoside for a long period of time to maintain required effective blood concentration for the therapeutic effect. Additionally, sustained release phase of the analgesic also enables the blood plasma concentration levels of the analgesic to be maintained above a minimum effective level and below its minimum toxic level in order to obtain the desired therapeutic effects in a reliable way when it is combined with said thiocolchicoside formulations.

Due to the use of glyceryl behenate as release rate controlling agent in the first sustained release phase, the plasma concentration level is made stable by maintaining the release of thiocolchicoside in the blood stream over an extended period of time sufficient to justify once or twice daily dosing and thus leads to increase patient compliance, and to prevent the fluctuation of thiocolchicoside concentration in the blood plasma that causes chemical imbalances when thiocolchicoside is combined with analgesic. In the pharmaceutical formulation of the present invention, the ratio of total weight of glyceryl behenate to the total weight of thiocolchicoside is between 25:1 to 1:25, preferably between 15:1 to 1:10, more preferably between 10:1 to 1:5 in the first sustained release phase.

According to present invention, the analgesic is preferably having low solubility in a medium such as water and selected from a group comprising diclofenac, indomethacin, etodolac, sulindac, tolmetin, aspirin, diflunisal, salsalate, ketorolac, ketoprofen, dexketoprofen, fenoprofen, oxaprozin, ibuprofen, flurbiprofen, naproxen, piroxicam, meloxicam, lornoxicam, morphine, metamizole, phenazone, aminopyrine, propyphenazone, phenylbutazone, nimesulide, nabumetone, bupivacaine, clonidine, hydromorphone, baclofen, fentanyil, buprenorphine, and sufentanil, or a pharmaceutically acceptable salts thereof. The analgesic is preferably selected from the group comprising flurbiprofen, ibuprofen, naproxen, fenoprofen, ketoprofen, nimesulide or a pharmaceutically acceptable salts thereof, the analgesic is more preferably flurbiprofen and a pharmaceutically acceptable salt thereof.

Another aspect of the present invention, the sustained release phase of the analgesic contained in the formulation of the invention, preferably comprises glyceryl behenate as release rate controlling agent to provide release of the analgesic over an extended period of the time for maintaining the plasma level of the analgesic.

Another aspect of the present invention, said pharmaceutical formulation may be administered orally, parenterally, ocularly, nasally, buccally, sublingually and topically. It is preferably administered by orally. Examples of oral dosage forms include tablets, multilayer tablets (coated or uncoated), multicoated tablets, capsules, hard or soft gelatin capsules, pellets, powders, granules, colloidal dispersions, dispersions, sterile solutions, suspensions, emulsions, etc.

Another aspect of the present invention, the pharmaceutical composition comprising the immediate release of thiocolchicoside and the first sustained release phase of thiocolchicoside and the second sustained release phase of an analgesic, in particular flurbiprofen, is used for the manufacture of a medicament for the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

Another aspect of the present invention, said pharmaceutical formulation may contain another release rate controlling agent besides glyceryl behenate selected from a group comprising glyceryl palmitostearte, glyceryl monostearate, polyglycolized glycerides, hydrogenated vegetable oils such as hydrogenated castor oil, microcrystalline wax, hydroxypropylmethylcellulose, hydroxypropylcellulose, cetyl alcohol, a polymethacrylate or mixtures thereof.

Another aspect of the invention, the pharmaceutical formulation further comprise a polymethacrylate, that is also identified as ammonio methacrylate copolymer, as a release rate controlling agent. According the present invention, ammonio methacrylate copolymer is preferably in aqueous dispersion form with low permeability and this aqueous dispersion contains a polymer dry weight content preferably between 10% and 50%, more preferably between 25% and 50%, most preferably of 30% (available under tradename Eudragit RS 30 D) based on the total weight of the aqueous dispersion.

During production of the pharmaceutical formulation according to the present invention, firstly, ammonio methacrylate copolymer is added in aqueous dispersion form. Howewer, after drying step of the production, water content of the aqueous dispersion is substantially lost, and the polymer dry weight content is remained in the final sustained release formulation. Therefore, the polymer dry weight content of ammonio methacrylate copolymer remained in the final formulation is between 0.5% and 15%, preferably between 1% and 12%, more preferably between 2% and 9% by weight based on the total weight of the final formulation.

It has found that when the ratio of the total weight of ammonio methacrylate copolymer aqueous dispersion to the total weight of glyceryl behenate is between 1:0.1 and 1:30 in the first sustained release phase, ammonio methacrylate copolymer aqueous dispersion helps glyceryl behenate to maintains the release of thiocolchicoside over a more extended period of time. The ratio of the total weight of ammonio methacrylate copolymer aqueous dispersion to the total weight of glyceryl behenate is preferably between 1:0.5 and 1:20, more preferably between 1:1 and 1:10 in the first sustained release phase.

Another aspect of the present invention, the pharmaceutical formulation is preferably in the form of a multilayer tablet comprising an immediate release phase comprising thiocolchicoside or a pharmaceutically acceptable salt thereof, first sustained release phase comprising thiocolchicoside or a pharmaceutically acceptable salt thereof and glyceryl behenate as release rate controlling agent, and second sustained release phase of an analgesic, particularly flurbiprofen, or a pharmaceutically acceptable salt thereof. Preferably, there is at least one barrier layered between the layers to separate them from each other.

It is know in the prior art that the surface area of the pharmaceutical dosage forms is one of the most important features for its dissolution. Another aspect of the invention, at least one layer such as a barrier layer (d, e, f) between the layers of the tablet is used in order to obtain a separation between both formulations easily, without damaging the surface areas *(**Figure 2 and 3**).* When this multilayer tablet contacts with the dissolution media they separate and act like two independent tablets. Therefore, the surface area of each layer is evaluated independently and dissolution rate of the active agents comprised by layers is increased since the contact area between surface of each layer and dissolution media is increased. Additionally, by means of barrier layers, the probable interactions between different formulations can be minimized.

The pharmaceutical formulation of the present invention is administered orally in the form of a tablet, multilayer tablet, multicoated tablet and capsule. This formulation can be prescribed once-a-day or twice-a-day to a patient in need thereof to deliver the drug over, approximately in 24 hours period.

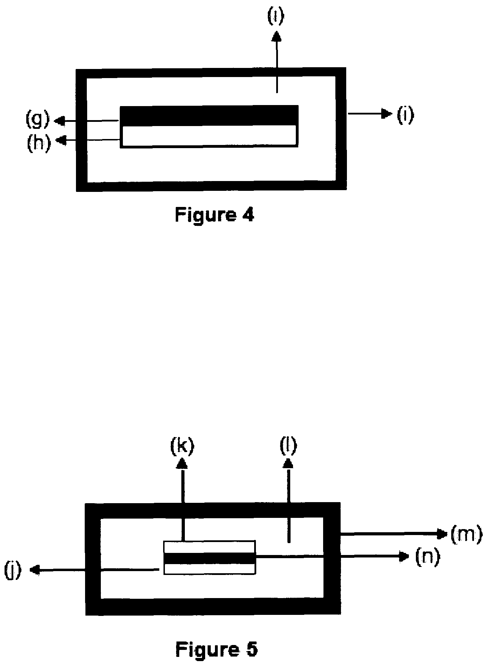

In the other preferred aspect, the pharmaceutical composition is in the form of a multicoated tablet *(**Figure 4**)* comprising:
- a core comprising two layers, one of which comprises the first sustained-release phase of thiocolchicoside and pharmaceutically acceptable excipients (g), and other layer comprises the second sustained-release phase of flurbiprofen and pharmaceutically acceptable excipients (h),
- a coating layer comprising thiocolchicoside which is released immediately (i),
- optionally a polymer coating layer including conventional coating polymers like Opadry® (i).

Another aspect of the present invention, the pharmaceutical composition is in the form of a multicoated tablet *(**Figure 5**)* comprising:
- a core comprising three layers, one of which comprises the first sustained-release phase of thiocolchicoside and pharmaceutically acceptable excipients (k), and other layer comprises the second sustained-release phase of flurbiprofen and pharmaceutically acceptable excipients (j), wherein there is also a barrier layer (n),
- optionally a polymer coating layer including conventional coating polymers like Opadry® (I),
- a coating layer comprising thiocolchicoside which is released immediately (m).

Another aspect of the present invention, the pharmaceutical formulation is in the form of a multicoated tablet comprising a core comprising the sustained relase pellets with immediate release granules and pharmaceutically acceptable excipients, preferably with a coating layer.

Another aspect of the present invention, an effective amount of thiocolchicoside or a pharmaceutically acceptable salt thereof and an effective amount of the analgesic or a pharmaceutically acceptable salt thereof can be present in the same sustained release phase that is also comprising glyseryl behenate as release rate controlling agent.

Another aspect of the present invention, the pharmaceutical formulation is in an orally administrable sustained release pharmaceutical dosage form for the patients in need of therapeutic relief from spasticity associated with, for example, degenerative vertebra diseases as torticollis, dorsalgy, lombalgy, disk hernia, neurologic and traumatic disorders; painful muscle spasms associated with static and functional disorders of vertebra or occurred in post-operations of osteoarthritis; pain and inflammatory symptoms associated with tissue trauma.

The pharmaceutical compositions of the invention include tablets, multilayer tablets, multicoated tablets and capsules which can be made in accordance with methods that are standard in the art.

According to the present invention, both of sustained release phase of thiocolchicoside or an analgesic and immediate release phase of thiocolchicoside further comprise at least one pharmaceutically acceptable excipient selected from a group comprising polymers, lubricants, binding agents, fillers, preservatives, disintegrants, plasticizers, aromatic substances, or a mixture thereof. Fillers selected from the group comprising starch, lactose (e.g. lactose monohydrate), microcrystalline cellulose, carboxy cellulose sodium, sucrose; binding agents selected from the group comprising povidone, hydroxypropylcellulose, hydroxypropylmethylcellulose (HPMC), ethylcellulose (EC), hydroxypropylmethylcellulose phthalate (HPMCP), hydroxyethylcellulose (HEC), methylcellulose (MC), sodium carboxymethylcellulose and cellulose acetate butyrate, sugar, starch (e.g. corn starch), gelatin (e.g. Gelatin 200 Bloom); lubricants selected from the group comprising collodial anhydrous silica, colloidal silicon dioxide, magnesium stearate, talc, sodium stearyl fumarate; disintegrants selected from the group comprising microcrystalline cellulose, sodium starch glycollate, croscarmellose sodium, crospovidone, starch and their mixtures; coating agents selected from the group comprising ethyl cellulose, polymethacrilates, triethyl citrate; plasticizer selected from the group comprising diethyl phthalate (DEP), triethyl citrate, polyethylene glycol; aromatic agents are selected from the group comprising fruit aromas such as of orange, cherry, strawberry, banana, sourcherry, lemon, etc.; aromas of cardamom, anis, mint, menthol, eucalyptus, vanillin, and ethyl vanillin, and the mixtures thereof; preservatives are selected from the group comprising methylparaben and propylparaben and the salts thereof (e.g. sodium, potassium), sodium benzoate, citric acid, benzoic acid, butylated hydroxytoluene and butylated hydroxyanisole, and the mixtures thereof.

Preferably, the pharmaceutical formulation of the invention is in the form of a multilayer tablet and/or multicoated tablet. According to the present invention it may be produced by any standard tabletting technique, e.g. by wet granulation, dry granulation or direct compression.

As mentioned above, this invention is comprising the active ingredients, an analgesic, in particular flurbiprofen, or a pharmaceutically acceptable salt thereof in combination with thiocolchicoside or a pharmaceutically acceptable salt thereof wherein the analgesic is present in an amount of between 50 and 500 mg and thiocolchicoside is present in an amount of between 2 and 25 mg, preferred embodiments of the analgesic is present in an amount of between 100 and 300 mg and thiocolchicoside is present in an amount of between 4 and 20 mg.

According to the present invention, the immediate release and the sustained release phase of thiocolchicoside have an amount of thiocolchicoside as follows: %1-25% of thiocolchicoside content of the pharmaceutical formulation of the invention is in the immediate release phase, the remaining 75%-99% of thiocolchicoside content of said pharmaceutical formulation is in the sustained release phase.

This invention is further defined by reference to the following examples. Although the examples are not intended to limit the scope of the present invention, it should be considered in the light of the description detailed above.

### Examples:

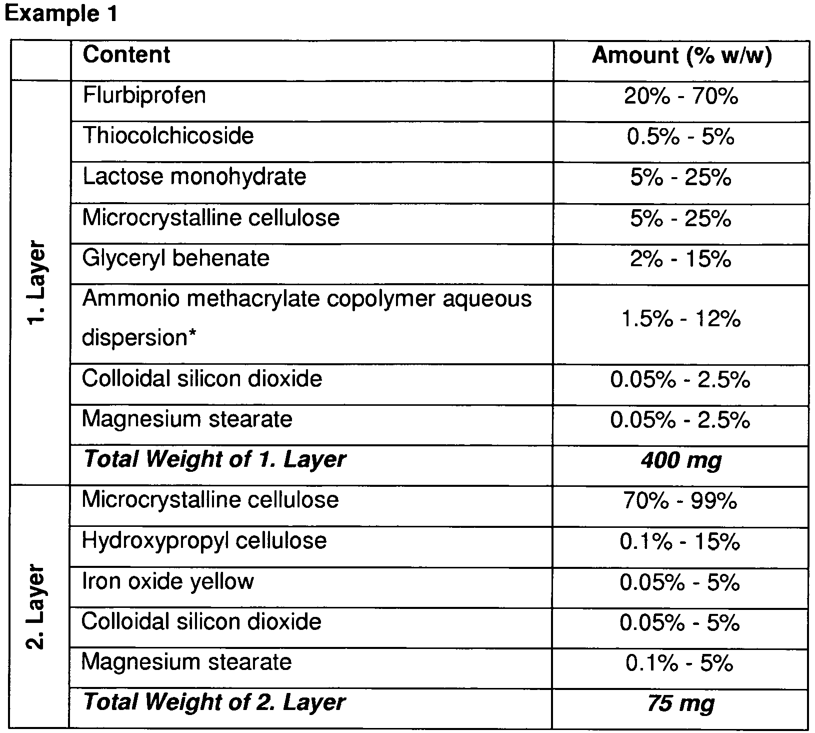

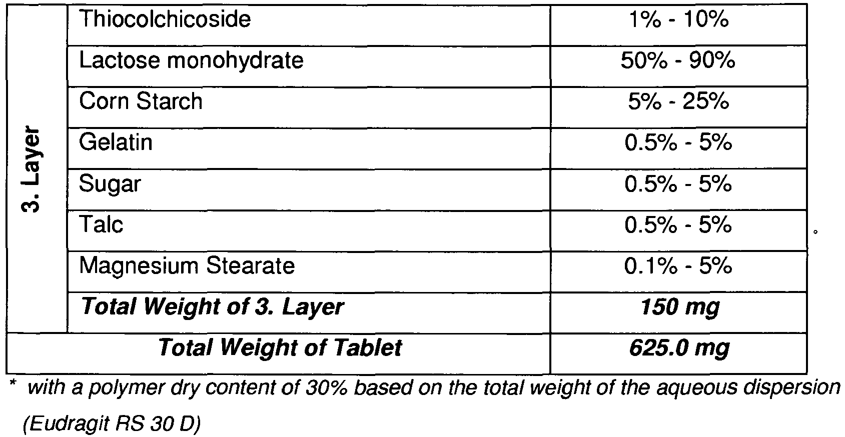

### Preparation of 1. Layer:

Flurbiprofen, Thiocolchicoside, lactose monohydrate, microcrystalline cellulose, glyceryl behenate, that are previously weighed, are taken into a high shear granulator and are mixed here. The powder mixture in the high shear granulator is granulated with ammonio methacrylate copolymer aqueous dispersion with a polymer dry weight content of 30% (under the tradename Eudragit RS 30 D). Then, this mixture is passed through Frewitt sieve with the pore size of 5 mm and dried in a fluid bed drier. After drying, it is passed through Frewitt sieve with the pore size of 1 mm. Colloidal silicon dioxide is added the mixture and they are mixed for 15 minutes. Finally, magnesium stearate is added to the mixture and they are mixed for 5 minutes.

### Preparation of 2. Layer:

A part of microcrystalline cellulose and hydroxypropyl cellulose, that are previously weighed, are taken into an intermediate bulk container. Iron oxide yellow, colloidal silicon dioxide and the remaining part of microcrystalline cellulose are passed through a sieve with the pore size of 630 µm and then taken into the intermediate bulk container. The powder mixture is mixed for 20 minutes with a SP 2000 Blender. Magnesium stearate is added to this mixture and then they are mixed for 5 minutes.

### Preparation of 3. Layer:

Thiocolchicoside, lactose monohydrate, corn starch and sugar, that are previously weighed, are taken into a high shear granulator, and then they are mixed. The aqueous solution of previously weighed-gelatin is prepared, and the powder mixture is granulated with this solution. The wet granules are grinded, and then dried in a drying oven until having moisture content of 3%. The dry granules are passed through a grinder and taken into a mixer. Talc is added to the granules and they are mixed for 5 minutes, and then magnesium stearate is added and they are for 3 minutes more.

All of the homogenous mixtures obtained from the preparations of 1. Layer, 2. Layer and 3. Layer are compressed to obtain a multilayered tablet *(**Figure 2**).*

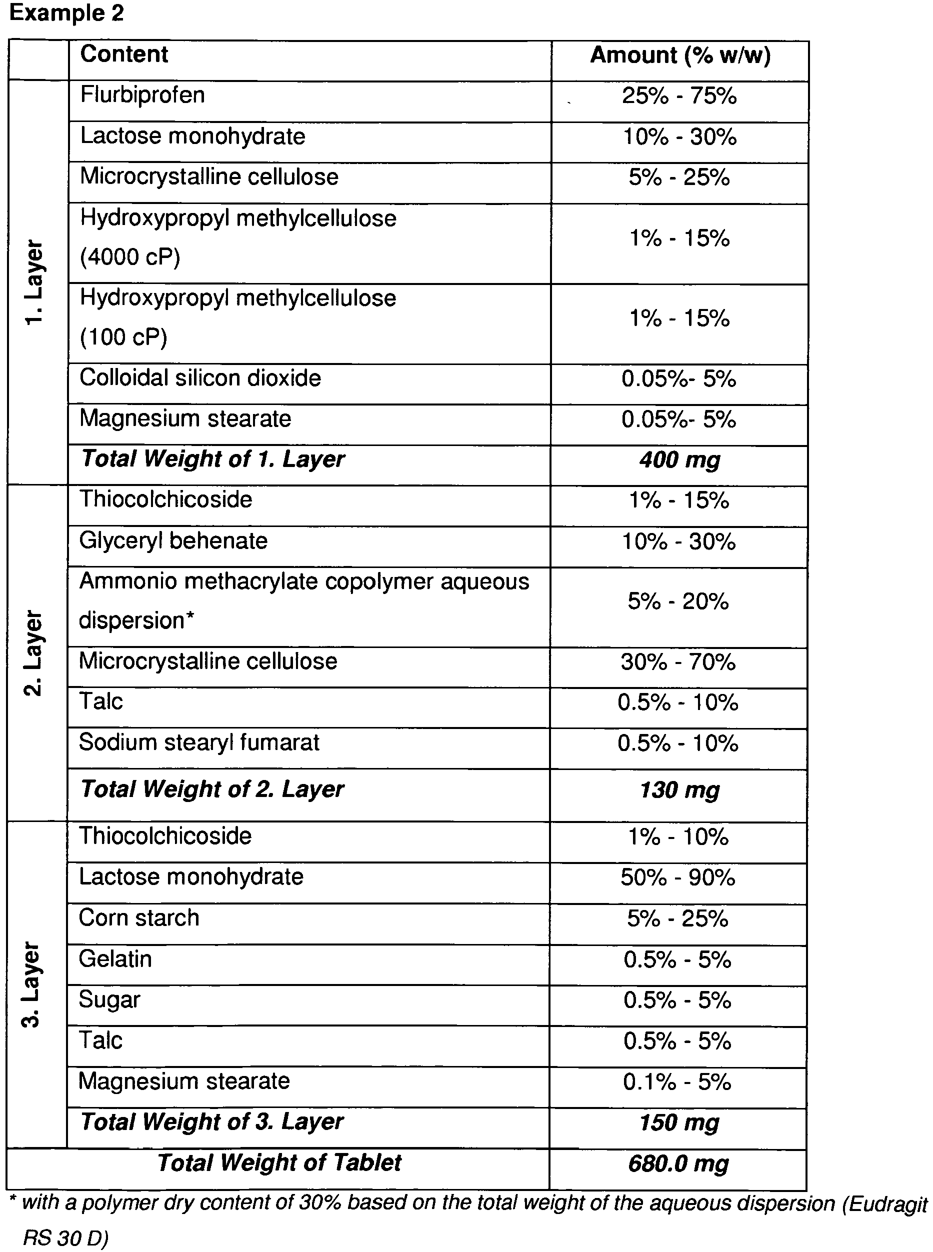

### Preparation of 1. Layer:

Flurbiprofen, lactose monohydrate, microcrystalline cellulose, hydroxypropyl methylcellulose (4000 cP), hydroxypropyl methylcellulose (100 cP) that are previously weighed, are taken into a high shear granulator and are mixed here. The powder mixture is wet granulated in the high shear granulator. Then, this mixture is passed through Frewitt sieve with the pore size of 5 mm and dried in a fluid bed drier. After drying, it is passed through Frewitt sieve with the pore size of 1 mm. Colloidal silicon dioxide is added to the mixture and they are mixed for 15 minutes. Finally, magnesium stearate is added to the mixture and they are mixed for 5 minutes.

### Preparation of 2. Layer:

Thiocolchicoside, glyceryl behenate and microcrystalline cellulose, that are previously weighed, are mixed to form a mixture. The obtained mixture is granulated with ammonio methacrylate copolymer aqueous dispersion with a polymer dry weight content of 30% (under the tradename Eudragit RS 30 D). The granules are sieved, then dried in a drying oven at the temperature of 40°C-60°C until having moisture content of 2% and milled until having a particle size of 700 µm. Talc is added and mixed with the granules for 5 minutes, then sodium stearyl fumarate is added and mixed with granules for 3 minutes

### Preparation of 3. Layer:

Thiocolchicoside, lactose monohydrate, corn starch and sugar, that are previously weighed, are taken into a high shear granulator, and then they are mixed. The aqueous solution of previously weighed-gelatin is prepared, and the powder mixture is granulated with this solution. The wet granules are grinded, and then dried in a drying oven until having moisture content of 3%. The dry granules are passed through a grinder and taken into a mixer. Talc is added to the granules and they are mixed for 5 minutes, and then magnesium stearate is added and they are for 3 minutes more.

All of the homogenous mixtures obtained from the preparations of 1. Layer, 2. Layer and 3. Layer are compressed to obtain a multilayered tablet *(**Figure 1**).*

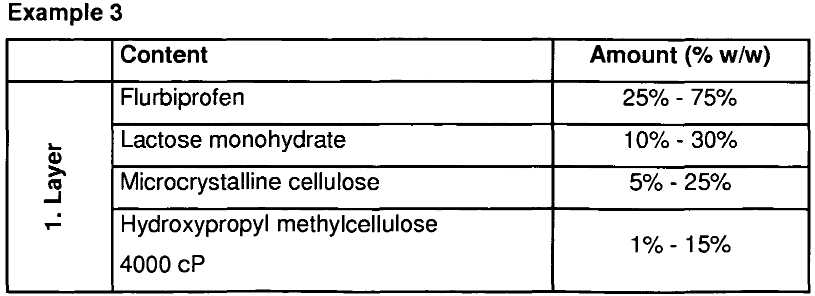

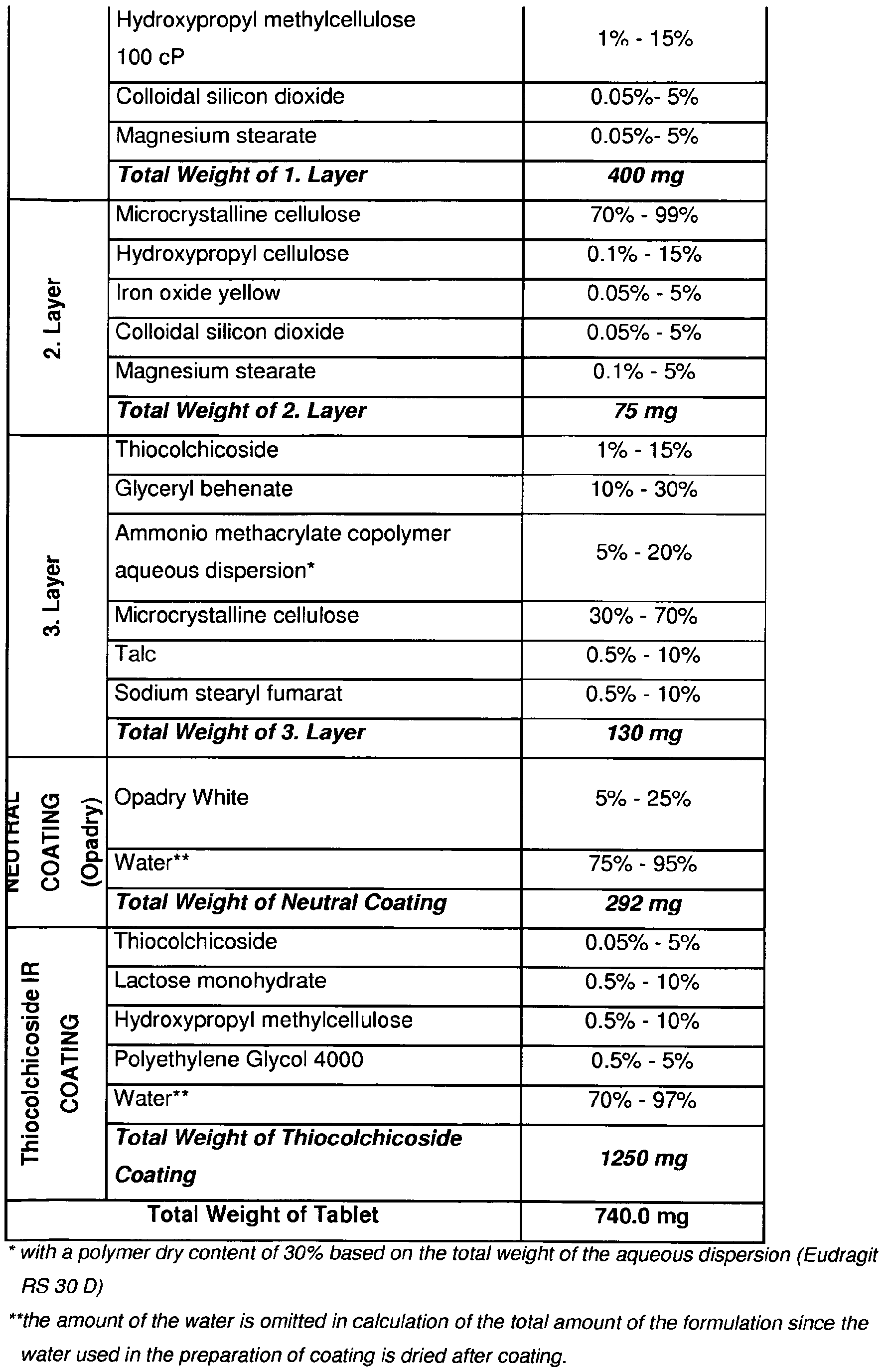

### Preparation of 1. Layer:

Flurbiprofen, lactose monohydrate, microcrystalline cellulose, hydroxypropyl methylcellulose (4000 cP), hydroxypropyl methylcellulose (100 cP) that are previously weighed, are taken into a high shear granulator and are mixed here. The powder mixture is wet granulated in the high shear granulator. Then, this mixture is passed through Frewitt sieve with the pore size of 5 mm and dried in a fluid bed drier. After drying, it is passed through Frewitt sieve with the pore size of 1 mm. Colloidal silicon dioxide is added to the mixture and they are mixed for 15 minutes. Finally, magnesium stearate is added to the mixture and they are mixed for 5 minutes.

### Preparation of 2. Layer:

A part of microcrystalline cellulose and hydroxypropyl cellulose, that are previously weighed, are taken into an intermediate bulk container. Iron oxide yellow, colloidal silicon dioxide and the remaining part of microcrystalline cellulose are passed through a sieve with the pore size of 630 µm and then taken into the intermediate bulk container. The powder mixture is mixed for 20 minutes with a SP 2000 Blender. Magnesium stearate is added to this mixture and then they are mixed for 5 minutes.

### Preparation of 3. Layer:

Thiocolchicoside, glyceryl behenate and microcrystalline cellulose, that are previously weighed, are mixed to form a mixture. The obtained mixture is granulated with ammonio methacrylate copolymer aqueous dispersion with a polymer dry weight content of 30% (under the tradename Eudragit RS 30 D). The granules are sieved, then dried in a drying oven at the temperature of 40°C-60°C until having moisture content of 2% and milled until having a particle size of 700 µm. Talc is added and mixed with the granules for 5 minutes, then sodium stearyl fumarate is added and mixed with granules for 3 minutes. All of the homogenous mixtures obtained from the preparations of 1. Layer, 2. Layer and 3. Layer are compressed to obtain a multilayered tablet.

### Preparation of Neutral Coating:

The aqueous solution of Opadry White is prepared and then the obtained multilayer tablets are film-coated with the aqueous solution of Opadry White.

### Preparation of Thiocolchicoside IR Coating:

For second coating, Thiocolchicoside, lactose monohydrate, hydroxypropyl methylcellulose and polyethylene glycol 4000, that are previously weighed, are added to water slowly and then are mixed with a propeller agitator at a rate that results in a vortex. The mixing is continued until a homogenous mixture is obtained. Finally, the tablets coated with neutral coating are secondly coated with this homogenous mixture *(**Figure 5**)*.

## Claims

1. A pharmaceutical formulation comprising:
- an immediate release phase comprising an effective amount of thiocolchicoside or a pharmaceutically acceptable salt thereof, and
- first sustained release phase comprising an effective amount of thiocolchicoside or a pharmaceutically acceptable salt thereof and glyceryl behenate as release rate controlling agent, and
- second sustained release phase comprising an effective amount of an analgesic or pharmaceutically acceptable salt thereof.

2. The pharmaceutical formulation according to claim 1, wherein the analgesic is selected from a group comprising diclofenac, indomethacin, etodolac, sulindac, tolmetin, aspirin, diflunisal, salsalate, ketorolac, ketoprofen, dexketoprofen, fenoprofen, oxaprozin, ibuprofen, flurbiprofen, naproxen, piroxicam, meloxicam, lornoxicam, morphine, metamizole, phenazone, aminopyrine, propyphenazone, phenylbutazone, nimesulide, nabumetone, bupivacaine, clonidine, hydromorphone, baclofen, fentanyil, buprenorphine, and sufentanil, or a pharmaceutically acceptable salts thereof.

3. The pharmaceutical formulation according to claim 2, wherein the analgesic is flurbiprofen.

4. The pharmaceutical formulation according to any of preceding claims, wherein the ratio of total weight of glyceryl behenate to the total weight of thiocolchicoside is between 25:1 to 1:25 in the first sustained release phase.

5. The pharmaceutical formulation according to claim 4, wherein the ratio of total weight of glyceryl behenate to the total weight of thiocolchicoside is between 15:1 to 1:10 in the first sustained release phase.

6. The pharmaceutical formulation according to claim 5, wherein the ratio of total weight of glyceryl behenate to the total weight of thiocolchicoside is between 10:1 to 1:5 in the first sustained release phase.

7. The pharmaceutical formulation according to any of preceding claims further comprising additional release rate controlling agent selected from a group comprising glyceryl palmitostearte, glyceryl monostearate, polyglycolized glycerides hydrogenated vegetable oils such as hydrogenated castor oil, microcrystalline wax, cetyl alcohol, a polymethacrylate or mixtures thereof.

8. The pharmaceutical formulation according to claim 7, wherein the additional release rate controlling agent is a polymethacrylate.

9. The pharmaceutical formulation according to claim 8, wherein polymethacrylate is ammonio methacrylate copolymer.

10. The pharmaceutical formulation according to claim 9, wherein ammonio methacrylate copolymer is in aqueous dispersion form.

11. The pharmaceutical formulation according to any of preceding claims, wherein the ratio of the total weight of ammonio methacrylate copolymer aqueous dispersion to the total weight of glyceryl behenate is between 1:0.1 and 1:30 in the first sustained release phase.

12. The pharmaceutical formulation according to claim 11, wherein the ratio of the total weight of ammonio methacrylate copolymer aqueous dispersion to the total weight of glyceryl behenate is between 1:0.5 and 1:20 in the first sustained release phase.

13. The pharmaceutical formulation according to claim 12, wherein the ratio of the total weight of ammonio methacrylate copolymer aqueous dispersion to the total weight of glyceryl behenate is between 1:1 and 1:10 in the first sustained release phase.

## Patentansprüche

1. Pharmazeutische Formulierung, umfassend:
- Eine Phase zur sofortigen Freisetzung, umfassend eine wirksame Menge an Thiocolchicosid oder ein pharmazeutisch akzeptables Salz davon, und
- eine erste Phase zur verzögerten Freisetzung, umfassend eine wirksame Menge an Thiocolchicosid oder eines pharmazeutisch akzeptablen Salzes davon und Glycerylbehenat als ein Mittel zur Kontrolle der Freisetzungsrate, und
- eine zweite Phase zur verzögerten Freisetzung, umfassend eine wirksame Menge eines Analgetikums oder eines pharmazeutisch akzeptablen Salzes davon.

2. Pharmazeutische Formulierung nach Anspruch 1, wobei das Analgetikum ausgewählt ist aus einer Gruppe, umfassend Diclofenac, Indomethacin, Etodolac, Sulindac, Tolmetin, Aspirin, Diflunisal, Salsalat, Ketorolac, Ketoprofen, Dexketoprofen, Fenoprofen, Oxaprozin, Ibuprofen, Flurbiprofen, Naproxen, Piroxicam, Meloxicam, Lornoxicam, Morphin, Metamizol, Phenazon, Aminopyrin, Propyphenazon, Phenylbutazon, Nimesulid, Nabumeton, Bupivacain, Clonidin, Hydromorphon, Baclofen, Fentanyil, Buprenorphin und Sufentanil oder ein pharmazeutisch akzeptables Salz davon.

3. Pharmazeutische Formulierung nach Anspruch 2, wobei das Analgetikum Flurbiprofen ist.

4. Pharmazeutische Formulierung nach einem der vorangehenden Ansprüche, wobei das Verhältnis des Gesamtgewichts an Glycerylbehenat zu dem Gesamtgewicht an Thiocolchicosid zwischen 25:1 zu 1:25 in der ersten Phase zur verzögerten Freisetzung beträgt.

5. Pharmazeutische Formulierung nach Anspruch 4, wobei das Verhältnis des Gesamtgewichts an Glycerylbehenat zum Gesamtgewicht an Thiocolchicosid zwischen 15:1 zu 1:10 in der ersten Phase zur verzögerten Freisetzung beträgt.

6. Pharmazeutische Formulierung nach Anspruch 5, wobei das Verhältnis des Gesamtgewichts an Glycerylbehenat zum Gesamtgewicht an Thiocolchicosid zwischen 10:1 zu 1:5 in der ersten Phase zur verzögerten Freisetzung beträgt.

7. Pharmazeutische Formulierung nach einem der vorangehenden Ansprüche, weiterhin umfassend ein zusätzliches Mittel zur Kontrolle der Freisetzungsrate, ausgewählt aus einer Gruppe, umfassend Glycerylpalmitostearat, Glycerylmonostearat, polyglycolisierte Glyceride, hydrierte pflanzliche Öle, wie hydriertes Rizinusöl, mikrokristallines Wachs, Cetylalkohol, ein Polymethacrylat oder Mischungen davon.

8. Pharmazeutische Formulierung nach Anspruch 7, wobei das zusätzliche Mittel zur Kontrolle der Freisetzungsrate ein Polymethacrylat ist.

9. Pharmazeutische Formulierung nach Anspruch 8, wobei das Polymethacrylat Ammonio-Methacrylat-Copolymer ist.

10. Pharmazeutische Formulierung nach Anspruch 9, wobei das Ammonio-Methacrylat-Copolymer in wässriger Dispersionsform vorliegt.

11. Pharmazeutische Formulierung nach einem der vorangehenden Ansprüche, wobei das Verhältnis des Gesamtgewichts an wässriger Dispersion von Ammonio-Methacrylat-Copolymer zum Gesamtgewicht an Glycerylbehenat zwischen 1:0,1 und 1:30 in der ersten Phase zur verzögerten Freisetzung beträgt.

12. Pharmazeutische Formulierung nach Anspruch 11, wobei das Verhältnis des Gesamtgewichts an wässriger Dispersion von Ammonio-Methacrylat-Copolymer zum Gesamtgewicht an Glycerylbehenat zwischen 1:0,5 und 1:20 in der ersten Phase zur verzögerten Freisetzung beträgt.

13. Pharmazeutische Formulierung nach Anspruch 12, wobei das Verhältnis des Gesamtgewichts an wässriger Dispersion von Ammonio-Methacrylat-Copolymer zum Gesamtgewicht an Glycerylbehenat zwischen 1:1 und 1:10 in der ersten Phase zur verzögerten Freisetzung beträgt.

## Revendications

1. Formulation pharmaceutique comprenant :
- une phase à libération instantanée d'une quantité efficace de thiocolchicoside ou d'un sel pharmaceutiquement acceptable de celui-ci, et
- une première phase à libération prolongée d'une quantité efficace de thiocolchicoside ou d'un sel pharmaceutiquement acceptable de celui-ci et de béhénate de glycéryle comme agent réglant la vitesse de libération, et
- une seconde phase à libération prolongée d'une quantité efficace d'un analgésique ou d'un sel pharmaceutiquement acceptable de celui-ci.

2. Formulation pharmaceutique suivant la revendication 1, dans laquelle l'analgésique est sélectionné parmi le groupe comprenant diclofénac, indométhacine, étodolac, sulindac, tolmétine, aspirine, diflunisal, salsalate, kétorolac, kétoprofène, dexkétoprofène, fénoprofène, oxaprozine, ibuprofène, flurbiprofène, naproxène, piroxicam, méloxicam, lornoxicam, morphine, métamizole, phénazone, aminopyrine, propyphénazone, phénylbutazone, nimesulide, nabumétone, bupivacaïne, clonidine, hydromorphone, baclofène, fentanyil, buprénorphine, et sufentanile ou des sels pharmaceutiquement acceptables de ceux-ci.

3. Formulation pharmaceutique suivant la revendication 2, dans laquelle l'analgésique est fluriprofène.

4. Formulation pharmaceutique suivant une quelconque des revendications précédentes, dans laquelle le rapport du poids total de béhénate de glycéryle au poids total de thiocolchicoside est compris entre 25:1 et 1:25 dans la première phase de libération prolongée.

5. Formulation pharmaceutique suivant la revendication 4, dans laquelle le rapport du poids total de béhénate de glycéryle au poids total de thiocolchicoside est compris entre 15:1 et 1:10 dans la première phase de libération prolongée.

6. Formulation pharmaceutique suivant la revendication 5, dans laquelle le rapport du poids total de béhénate de glycéryle au poids total de thiocolchicoside est compris entre 10:1 et 1:5 dans la première phase de libération prolongée.

7. Formulation pharmaceutique suivant une quelconque des revendications précédentes, comprenant, en outre, un agent réglant la vitesse de libération supplémentaire sélectionné parmi un groupe comprenant palmitostéarate de glycéryle, monostéarate de glycéryle, des glycérides polyglycolisés, des huiles végétales hydrogénées, telles que huile de ricin hydrogénée, cire microcristalline, alcool cétylique, un polyméthacrylate ou des mélanges de ceux-ci.

8. Formulation pharmaceutique suivant la revendication 7, dans laquelle l'agent réglant la vitesse de libération supplémentaire est un polyméthacrylate.

9. Formulation pharmaceutique suivant la revendication 8, dans laquelle le polyméthacrylate est un copolymère ammonio-méthacrylate.

10. Formulation pharmaceutique suivant la revendication 9, dans laquelle le copolymère ammonio-méthacrylate est sous forme de dispersion aqueuse.

11. Formulation pharmaceutique suivant une quelconque des revendications précédentes, dans laquelle le rapport du poids total de dispersion aqueuse de copolymère ammonio-méthacrylate au poids total de béhénate de glycéryle est compris entre 1:0,1 et 1:30 dans la première phase de libération prolongée.

12. Formulation pharmaceutique suivant la revendication 11, dans laquelle le rapport du poids total de dispersion aqueuse de copolymère ammonio-méthacrylate au poids total de béhénate de glycéryle est compris entre 1:05 et 1:20 dans la première phase de libération prolongée.

13. Formulation pharmaceutique suivant la revendication 12, dans laquelle le rapport du poids total de dispersion aqueuse de copolymère ammonio-méthacrylate au poids total de béhénate de glycéryle est compris entre 1:1 et 1:10 dans la première phase de libération.
